# DEMANDE DE BREVET EUROPEEN

(11) **EP 1 074 223 A1**
(43) Date de publication de la demande: **07.02.2001**
(21) Numéro de dépôt: 99122543.4
(22) Date de dépôt: 12.11.1999
(51) Int. Cl.: A61B 10/00

(54) **Dispositif de prelevement de cellules au niveau du col de l'uterus**

(30) Priorité: 03.08.1999 CH 142499
(71) Demandeur: Parvex Michel, 3232 Ins (DE); Kubler, Pierre-Aimé, 2000 Neuchatel (CH)
(72) Inventeur: Parvex Michel, 3232 Ins (DE); Kubler, Pierre-Aimé, 2000 Neuchatel (CH)
(74) Mandataire: Gresset, Jean

(57) **Abrégé**

Dispositif d'examen vaginal comportant un manche (10), de forme générale cylindrique et qui définit un axe (A-A), et un ensemble d'outils d'examen solidaires de ce manche, ledit ensemble comprenant
- une spatule (12) pour prélèvement de cellules de l'exocol et de la jonction squamo-cylindrique, et
- une brosse (16) endocervicale.

L'ensemble comprend, en outre, une tige pour col sténosé (14), venue de matière avec ledit manche (10), et en ce que l'un des autres outils (16) est formé d'une pièce rapportée, fixée par enclenchement sur ledit manche (10), et munie d'un trou (52) dans lequel ladite tige (14) est engagée.

## Description

La présente invention se rapporte à un dispositif d'examen vaginal, du type comportant un manche et un ensemble d'outils d'examen solidaires de ce manche. Cet ensemble comporte une spatule, pour le prélèvement de cellules de l'exocol et de la jonction squamo-cylindrique, et une brosse, pour le prélèvement de cellules dans la partie endocervicale du col de l'utérus. Un dispositif de ce type est décrit dans le document WO 93/11708. Il permet d'effectuer des examens vaginaux chez la plupart des patientes. Pourtant un cinquième d'entre elles environ a un col sténosé, qui empêche d'effectuer un prélèvement de cellules au moyen de la brosse endocervicale. Il est, de la sorte, nécessaire de prévoir d'autres outils pour ces patientes. Cela complique l'examen et augmente les coûts.

La présente invention a pour but principal de permettre au médecin effectuant un tel examen de le poursuivre sans devoir faire appel à de nouveaux moyens, même lorsque la patiente examinée a un col sténosé. Ce but est atteint grâce au fait que l'ensemble des outils comprend, en outre, une tige pour col sténosé, venue de matière avec le manche, et que l'un des autres outils est formé d'une pièce rapportée, fixée par enclenchement sur le manche, et munie d'un trou dans lequel la tige est engagée.

Le prélèvement de cellules se fait en tournant le dispositif dans le vagin. Il est bien clair que cela n'est possible que si la pièce rapportée est également entraînée en rotation. Il est, en outre, indispensable qu'au cours de l'examen, elle reste solidaire du manche. A cet effet, le manche et la pièce rapportée comportent des moyens de fixation et de positionnement complémentaires, agencés de manière à empêcher une rotation et une translation relative lors des opérations d'examen.

De manière avantageuse, l'outil rapporté est la brosse.

Avant d'entreprendre un prélèvement, le médecin commence par ouvrir le vagin au moyen d'un spéculum. Il peut ainsi l'examiner de visu. Cela lui permet ensuite de contrôler que les outils utilisés sont correctement positionnés. Malheureusement, la forme de ces outils peut empêcher une bonne vision. C'est le cas, par exemple, de la brosse décrite dans le document mentionné plus haut, qui comporte un tigeron axial, destiné à être engagé dans le col de l'utérus, et des bras s'étendant radialement et qui masquent le tigeron. De la sorte, le médecin ne peut pas travailler de manière précise, ce qui est désagréable voire douloureux pour la patiente et peut conduire à un prélèvement de cellules sur l'exocol uniquement.

Pour pallier cet inconvénient, la brosse selon l'invention est formée d'un tigeron, disposé dans le prolongement du manche, sur lequel des poils sont disposés radialement, dans un alignement hélicoïdal. De la sorte, le médecin peut parfaitement voir le travail qu'il effectue. En outre, la structure hélicoïdale favorise à la fois l'intromission de la brosse et le prélèvement de cellules.

Afin de faciliter l'introduction de la brosse dans le col de l'utérus, le tigeron est glabre sur au moins 20% de sa longueur dans son extrémité libre.

Dès lors que la brosse ne comporte pas de bras radiaux, le prélèvement effectué avec la brosse se limite à l'endocol. Pour effectuer un prélèvement de cellule provenant de l'exocol et de la jonction squamo-cylindrique la spatule comporte deux faces sensiblement parallèles et une tranche. Elle a une forme de coeur asymétrique avec un premier lobe de forme générale triangulaire isocèle et dont la pointe, arrondie, est décalée par rapport à l'axe du dispositif, et un second lobe défini par un demi-cercle, décalé dans la direction opposée au premier lobe. La partie de la tranche comprise entre les extrémités des deux lobes forme un bord de raclage avec un arrondi à leur intersection. Une telle forme ne masque pas la vision du médecin et assure un prélèvement sûr de cellules.

Le prélèvement de cellules dans un col sténosé doit se faire au moyen d'un outil de faibles dimensions, dont la forme ne blesse pas le col et qui assure un prélèvement optimal de cellules. Il doit, en outre, être suffisamment rigide pour qu'il ne fléchisse pas lors de son introduction. Ces conditions ont pu être remplies avec une tige s'inscrivant dans un tronc de cône dont la base est attenante au manche et de section en croix.

De manière particulièrement avantageuse, la croix comporte deux bras de longueurs inégales et la tige comprend, disposés axialement, des secteurs séparés par une paroi mince circulaire, deux secteurs voisins ayant leurs bras alignés, un bras long succédant à un bras court.

Pour que l'examen soit sûr, il est nécessaire que le maximum de cellules raclées reste attaché à l'outil. A cet effet, la surface des outils présente une structure granuleuse et comporte des alvéoles. De la sorte, les cellules restent dans les creux de la structure granuleuse, mais surtout dans le fond des alvéoles.

Afin d'augmenter encore le volume de cellules prélevées, la spatule comporte des trous passant d'une face à l'autre, dans le voisinage du bord de raclage.

Après prélèvement, le médecin doit pouvoir rapidement placer l'outil utilisé dans un lieu préservé afin que le prélèvement ne soit pas pollué. Dans ce but, le manche comporte, au voisinage de ses extrémités, une gorge définissant un point de rupture pour permettre de séparer facilement les outils du manche.

Pour effectuer un examen qui soit précis et aussi confortable que possible pour la patiente, il est nécessaire que l'outil puisse être tourné facilement, mais sans à-coup. La pratique a montré que les conditions étaient particulièrement favorables lorsque le manche comporte des cannelures arrondies qui définissent des cercles inscrits et circonscrits ayant des diamètres dans un rapport compris 80 et 90%.

D'autres avantages et caractéristiques de l'invention ressortiront de la description qui va suivre, faite en regard du dessin annexé, dans lequel:
- Les figures 1 et 2 sont des vues d'ensemble, de face et de profil, d'un dispositif selon l'invention, dans lequel l'outil rapporté est retiré de dessus l'outil solidaire du manche;
- La figure 3 est une coupe du manche, selon la ligne III-III de la figure 1;
- Les figures 4 et 5 représentent à une échelle agrandie et selon des vues orthogonales, un premier mode de réalisation d'une tige pour cols sténosés;
- Les figures 6 et 7 montrent un deuxième mode de réalisation de la tige, respectivement vue en plan et en coupe selon la ligne VII-VII de la figure 6;
- La figure 8 est une vue agrandie en plan et la figure 9 une coupe selon la ligne IX-IX de la figure 8, d'un premier mode de réalisation de spatule;
- Les figures 10 et 11 représentent, de manière similaire aux figures 8 et 9, un deuxième mode de réalisation de spatule; et
- La figure 12 est une vue agrandie et partiellement coupée d'une brosse selon l'invention.

Le dispositif représenté au dessin comporte un manche 10 et des outils, plus particulièrement une spatule 12, une tige 14 et une brosse 16.

Le manche 10, la spatule 12 et la tige 14 sont d'une seule pièce en plastique injecté, par exemple du polypropylène, choisi de manière qu'il présente une bonne rigidité, par exemple le produit vendu par la maison Montell Bayreuth Chemie Gmbh, Eschborn (Allemagne).

Le manche 10a une forme générale cylindrique, qui définit un axe A-A appelé axe du dispositif. Sa longueur est comprise entre 150 et 200 mm. Il comporte cinq secteurs, soit un secteur central 18, deux secteurs d'extrémités 20 et 22 au voisinage de la spatule 12, le secteur 20 étant attenant à la spatule, et deux secteurs d'extrémité 24 et 26 au voisinage de la tige 14, le secteur 24 étant attenant à la tige.

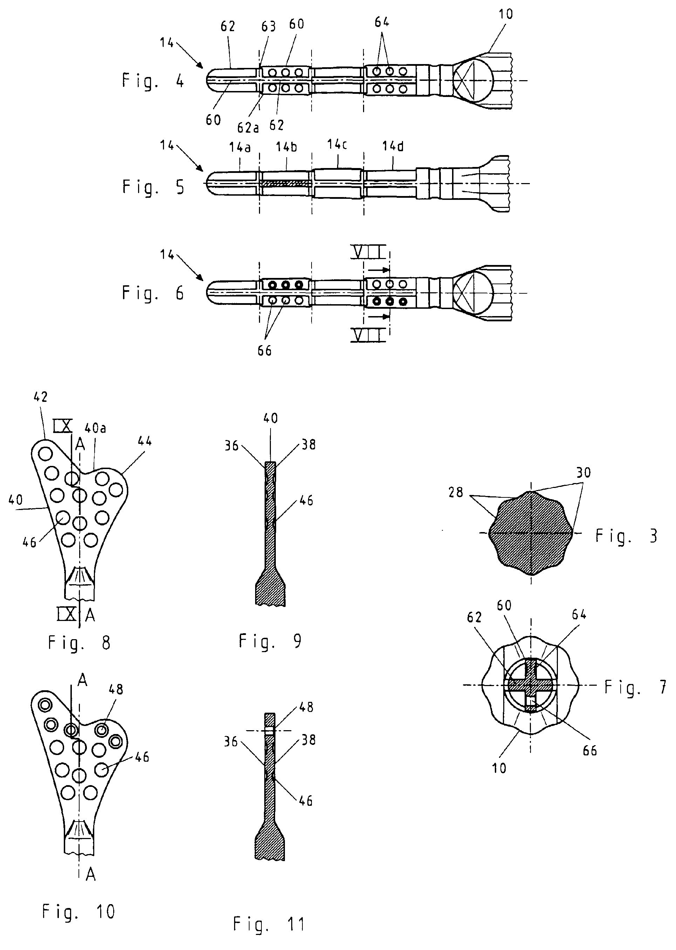

Comme le montre la figure 3, le secteur central 18 a une structure cannelée, formée d'une alternance de sillons 28 et de bossages 30 orientés parallèlement à l'axe A-A et répartis uniformément sur le pourtour, un bossage et un sillon embrassant ensemble un angle de 45°. Ces derniers sont définis par des arcs de cercles tangents les uns aux autres, pour former une structure sans arête. Le diamètre du cercle circonscrit, qui embrasse les bossages 30, est avantageusement compris entre 4 et 5 mm. Le diamètre du cercle inscrit, tangent au fond des sillons 28, est de 0,4 à 0,8 mm plus faible. En d'autres termes, les cercles inscrit et circonscrit ont des diamètres dans un rapport sensiblement compris 80 et 90%.

Les secteurs d'extrémité 20, 22, 24 et 26 ont également une structure cannelée, avec des sillons 28 et des bossages 30, mais de diamètre légèrement supérieur à celui du secteur central. Plus précisément, les diamètres des cercles inscrit et circonscrit de ces secteurs sont respectivement supérieurs aux diamètres des cercles inscrit et circonscrit du secteur central 18 de 0,5 mm à 1 mm. La structure étant en tout point comparable à celle du secteur 18, elle n'a pas été représentée en coupe.

Les secteurs d'extrémité 20 et 22 sont séparés par une gorge 32 définissant un point de rupture. Ainsi, après avoir effectué un prélèvement, le médecin peut séparer la spatule 12 du manche 10 par une simple flexion. La spatule 12, reste attachée au secteur d'extrémité 20, ce qui facilite sa manipulation, notamment pour la placer dans un récipient contenant un liquide de conservation avant que le prélèvement soit analysé. Le secteur 22 reste, par contre, solidaire du secteur central 18.

Les secteurs 24 et 26 sont, de même, séparés par une gorge 34, permettant de détacher la tige 14 du manche 10, pour la mettre à l'abri après prélèvement, par exemple dans le même récipient que la spatule.

Pour effectuer des prélèvements de cellules, le dispositif comporte donc trois outils, soit la spatule 12, la tige 14 et la brosse 16. La spatule 12 est agencée de manière à effectuer un prélèvement dans l'exocol. Ce type de prélèvement ne pose pas de problème particulier.

La brosse 16 assure le prélèvement dans l'endocol. Avec la plupart des patientes cette opération se fait sans problème. Toutefois, pour certaines, leur col est sténosé, de sorte que l'introduction de la brosse est douloureux et peut même provoquer des lésions. Dans de telles circonstances, et grâce au dispositif selon l'invention, le médecin peut effectuer un prélèvement au moyen de la tige 14, dont le diamètre est sensiblement plus faible que celui de la brosse 16, simplement en retirant cette dernière du manche 10, comme cela sera expliqué plus loin.

Pour mieux comprendre les problèmes relatifs à cet examen, les trois outils seront décrits de manière plus détaillée ci-après.

La spatule 12 comporte deux faces planes 36 et 38, sensiblement parallèles et une tranche 40 (Fig. 8 et 9). Elle a une épaisseur comprise entre 1 et 2 mm et une largeur comprise entre 15 et 20 mm. La longueur est de l'ordre de 25 mm. Elle présente, vue de face, une forme de coeur déformé, définie par deux lobes 42 et 44 disposés de part et d'autre de l'axe A-A et occupant des largeurs sensiblement égales.

Le lobe 42 est de forme générale triangulaire isocèle, avec un angle au sommet d'environ 30° et dont la pointe est arrondie. Le lobe 44 est défini par un demi-cercle dont le diamètre à la base fait un angle d'environ 45° avec l'axe A-A. La partie de la tranche 40 comprise entre les extrémités des deux lobes 42 et 44 forme un bord de raclage 40a, avec un arrondi concave à l'intersection des deux lobes. Le lobe 42 est destiné à assurer le positionnement de l'outil, celui-ci étant engagé dans le col de l'utérus. Le bord de raclage 40a est agencé de manière à être en contact avec l'exocol et la jonction squamo-cylindrique.

Pour faire adhérer autant de cellules que possible à la spatule lors du prélèvement, les faces 36 et 38 comportent entre dix et quinze creusures ayant forme de calottes sphériques, qui définissent des alvéoles 46 occupant environ un tiers de la surface de chaque face. La profondeur des alvéoles est de l'ordre du quart de l'épaisseur de la spatule.

Dans le mode de réalisation représenté aux figures 10 et 11, les alvéoles voisines du bord de raclage 40a sont remplacées par des trous 48, biseautés, dont le diamètre est sensiblement égal à l'épaisseur de la spatule. Cette solution permet d'augmenter encore le volume de prélèvement.

La brosse 16 est fabriquée par injection de polyéthylène, par exemple le produit connu sous la dénomination PE-LD Riblene MP 30, commercialisé par la maison Enichem Polymères, Mazingarbe (France). Ce matériau confère à la brosse une grande souplesse, de sorte qu'elle épouse au mieux les formes du col. En outre, cela permet d'éviter des désagréments à la patiente.

La brosse 16 comporte un capuchon 50, de longueur légèrement supérieure à celle de la tige 14 et définissant un logement 52 dans lequel se trouve cette dernière. L'extrémité ouverte est engagée sur l'extrémité du secteur 24. Elle est agencée de manière à former des moyens 53 de positionnement de la brosse 16 sur la tige 14. Plus précisément, pour assurer le positionnement axial, la paroi du logement 52 comporte un bossage 53a annulaire et l'extrémité du secteur 24 proche de la tige 14 une rainure 53b, dans laquelle le bossage 53a est engagé. Le positionnement angulaire est assuré par deux protubérances 53c diamétralement opposées, formées dans le capuchon 50, plus précisément dans la paroi du logement 52 au voisinage de son ouverture, coopérant avec deux arrondis concaves 53d formés dans le secteur 24, et reliant la partie cannelée à la zone comportant la rainure 53b.

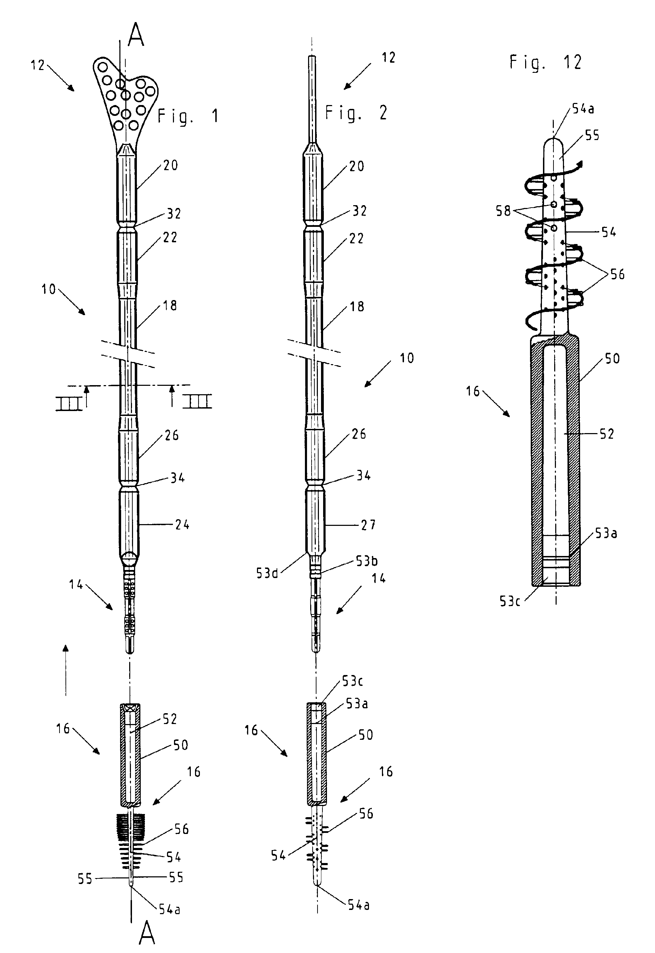

L'extrémité fermée du capuchon porte un tigeron 54, de forme tronc conique, avec un angle au sommet de l'ordre de 1 à 2° tronqué par deux plans parallèles 55 disposés symétriquement par rapport à l'axe A-A (fig 2). Le diamètre de la base du tronc de cône, adjacente au capuchon, est de l'ordre de 3 mm. Le tigeron 54 a une longueur comprise entre 20 et 25 mm. Il est muni de poils 56 tant sur ses côtés arrondis que sur ses faces planes, la densité étant sensiblement plus forte sur ces dernières.

Les poils 56 des côtés arrondis ont une longueur de l'ordre de 2 mm alors que celle des poils portés par les faces planes va décroissante de la base vers la pointe, passant de 3 à 2 mm.

De manière avantageuse et comme pour la spatule 12, les parois du tigeron 14 ont une structure granuleuse et comporte des alvéoles 58 (fig 12).

On relèvera que l'extrémité libre 54a du tigeron 54 ne comporte pas de poil. Il est, de la sorte, plus facile de l'introduire dans le col de l'utérus de la patiente.

La tige 14, venue de matière avec le manche 10 a une longueur de l'ordre de 25 mm. Elle s'inscrit dans un tronc de cône dont la base a un diamètre de l'ordre de 3 mm et le sommet de 2 mm, l'extrémité étant arrondie. Sa section est en croix, avec deux bras 60 et 62 orthogonaux, qui se coupent en leur milieu, les bras 60 étant plus long d'environ 0.2 mm que les bras 62. La tige 14 comporte, axialement, quatre secteurs 14a à 14d séparés par une paroi mince circulaire 63, dont le diamètre est sensiblement égal à la longueur des bras courts 62 (Fig. 5). Dans ces secteurs, les bras sont alignés, un bras long 60 succédant à un bras court 62. Les extrémités 60a des bras longs 60 sont arrondies à la liaison avec les parois 63, de manière à éviter qu'elles ne blessent la patiente lors de l'introduction de la tige 14.

Comme pour les autres outils, la surface de la tige 14 présente une structure granuleuse, certains bras étant, en outre, munis d'alvéoles 64, ainsi qu'on peut le voir sur les figures 4 et 5. Dans la variante représentée aux figures 6 et 7, une partie des alvéoles est remplacée par des trous 66.

Les alvéoles 64, les trous 66 et la structure granuleuse ont pour fonction d'assurer le maintien d'un maximum de cellules sur l'outil. Les essais effectués ont montré qu'il était avantageux d'avoir une structure granuleuse sur toute la surface de la tige 14 et de munir deux des quatres bras longs 60 d'alvéoles 64 ou de trous 66.

Il est évident que le dispositif décrit peut comporter de nombreuses variantes sans pour autant sortir du cadre de l'invention. Ainsi, les formes des outils peuvent être considérablement modifiées. Il serait également possible de prévoir une spatule 12 amovible, comportant un logement dans lequel serait logé la tige 14. Cette solution est toutefois moins intéressante, car l'examen se fait soit avec la brosse 16, soit avec la tige 14. De la sorte, l'examen serait effectué avec les deux outils disposés d'un même côté du manche. Il est, de plus, souhaitable que la brosse soit réalisée en un matériau souple de manière à rendre l'examen aussi confortable que possible. Cela serait plus difficile à réaliser si elle était de même matériau que le manche 10.

## Revendications

1. Dispositif d'examen vaginal comportant un manche (10), de forme générale cylindrique et qui définit un axe (A-A), et un ensemble d'outils d'examen solidaires de ce manche, ledit ensemble comprenant
• une spatule (12) pour prélèvement de cellules de l'exocol et de la jonction squamo-cylindrique, et
• une brosse (16) endocervicale,
caractérisé en ce que ledit ensemble comprend, en outre, une tige pour col sténosé (14), venue de matière avec ledit manche (10), et en ce que l'un des autres outils (16) est formé d'une pièce rapportée, fixée par enclenchement sur ledit manche (10), et munie d'un trou (52) dans lequel ladite tige (14) est engagée.

2. Dispositif selon la revendication 1, caractérisé en ce que ledit manche (10) et ladite pièce rapportée (16) comportent des moyens de fixation et de positionnement complémentaires , agencés de manière à empêcher une rotation et une translation relative lors des opérations d'examen.

3. Dispositif selon l'une des revendications 1 et 2, caractérisé en ce que l'outil rapporté est la brosse (16).

4. Dispositif selon la revendication 3, caractérisé en ce que ladite brosse (16) est formé d'un tigeron (54) , disposé dans le prolongement du manche, sur lequel des poils (56) sont disposés radialement, dans un alignement hélicoïdal.

5. Dispositif selon l'une des revendications 3 et 4, caractérisé en ce que ledit tigeron (54) est glabre sur au moins 20% de sa longueur dans son extrémité libre (54a).

6. Dispositif selon l'une des revendications 1 à 5, caractérisé en ce que ladite spatule (12) comporte deux faces (36, 38) sensiblement parallèles et une tranche (40) ayant une forme de coeur asymétrique avec un premier lobe (42) de forme générale triangulaire isocèle et dont la pointe, arrondie, est décalée par rapport à l'axe (A-A) du dispositif, et un second lobe (44) défini par un demi-cercle, décalé dans la direction opposée au premier lobe, les deux lobes étant reliés par un arrondi dont le centre se trouve sensiblement dans ledit axe, le tout agencé de manière que la partie de la tranche comprise entre les extrémités des deux lobes forme un bord de raclage (40a) avec un arrondi à l'intersection des deux lobes.

7. Dispositif selon l'une des revendications 1 à 6, caractérisé en ce que ladite tige (14) s'inscrit dans un tronc de cône dont la base est attenante au manche (10) et de section en croix.

8. Dispositif selon la revendication 7, caractérisé en ce que ladite croix est définie par deux bras de longueurs inégales (60, 62), se croisant en leur milieu, et en ce que ladite tige (14) comporte, selon une orientation axiale, une pluralité de secteurs (14a, 14b, 14c, 14d) dans lesquels les bras (60, 62) sont alignés, un bras long (60) succédant à un bras court (62).

9. Dispositif selon l'une des revendications 1 à 8, caractérisé en ce que les surfaces desdits outils (12, 14, 16) présentent une structure granuleuse et comportent des alvéoles (46, 58, 66).

10. Dispositif selon les revendications 6 et 9, caractérisé en ce que la spatule (12) comporte des trous (48) passant d'une face à l'autre dans le voisinage du bord de raclage (40a).

11. Dispositif selon l'une des revendications 1 à 10, caractérisé en ce que ledit manche (10) comporte, au voisinage de ses extrémités, une gorge (32, 34) définissant un point de rupture pour permettre de séparer facilement les outils (12, 14) du manche 10.

12. Dispositif selon l'une des revendications 1 à 11, caractérisé en ce que ledit manche (10) comporte des cannelures arrondies (28, 30) qui définissent des cercles inscrit et circonscrit ayant des diamètres dans un rapport compris 80 et 90%.
